# EUROPEAN PATENT APPLICATION

(11) **EP 3 795 673 A1**
(43) Date of publication of application: **24.03.2021**
(21) Application number: 19803909.1
(22) Date of filing: 15.05.2019
(51) Int. Cl.: C12M 3/00, C12N 5/077

(54) **CELL SCAFFOLD MATERIAL**

(30) Priority: 16.05.2018 JP 2018094922
(71) Applicant: Stem Cell & Device Laboratory, Inc., Kyoto-shi, Kyoto 600-8491 (JP)
(72) Inventor: HORI Kosuke, Kyoto-shi Kyoto 600-8491 (JP); KAWAHARA Noriyuki, Kyoto-shi Kyoto 600-8491 (JP)
(74) Representative: Herrero & Asociados, S.L.
(86) International application number: PCT/JP2019/019285
(87) International publication number: WO 2019/221172

(57) **Abstract**

**[Problem]** To provide a fiber sheet that can be used as a cell scaffold material and provide a stable and uniform cell sheet.

[Solution] The present invention provides an oriented fiber sheet. The diameter, in an orthogonal cross-section, of a fiber forming the fiber sheet is 1 µm to 7 µm. The pitch of the fiber sheet is 6 µm to 60 µm, the porosity is 10% to 60%, and the thickness is 4 µm to 70 µm. When a plurality of layers of the fiber sheet are stacked upon one another, the orientation axes of the fiber sheets intersect at angles of 5° to 25°. The fiber forming the fiber sheet are prepared from a biodegradable or non-biodegradable polymer material. PLGA is preferable as a biodegradable polymer material. Polystyrene is preferable as a non-biodegradable polymer material.

## Description

### TECHNICAL FIELD

The present invention relates to an oriented fiber sheet and a production method thereof. Specifically, the present invention relates to a cell scaffold material comprising an oriented fiber sheet for culturing cells and a production method thereof. Moreover, the present invention relates to a cell sheet obtained by culturing cells with the fiber sheet.

### BACKGROUND ART

In order to efficiently culture cells, various means of providing three-dimensional scaffolds to which cells adhere to promote cell proliferation have been reported. For example, a cell scaffold material composed of nanofibers composed of polyolefin, polyamide, polyurethane, polyester, fluorine-based polymer, polylactic acid, polyvinyl alcohol and the like, or a cell scaffold material composed of the nanofiber to which protein components are adsorbed is used to effectively perform cell culture or tissue regeneration (patent document 1). A cell scaffold material having a hollow fiber membrane mesh and a nanofiber layer is used in three-dimensional cell culture. Thus, the supply of nutrients and oxygen to the cultured cells and the removal of the metabolite waste from the cultured cells are performed with high efficiency (patent document 2). A large amount of pluripotent stem cells is supplied and cell death is suppressed by using a cell scaffold material composed of a nanofiber containing gelatin, collagen or cellulose, or a crosslinked nanofiber (patent document 3). A cell scaffold material obtained by using polyglycolic acid as a support and coated with nanofibers composed of polyglycolic acid, gelatin or the like is used to improve the proliferation rate of human pluripotent stem cells (patent document 4).

In recent years, treatment for transplanting sheet-like cultured myocardial cells to a disease part is attempted in patients with severe heart failure caused by ischemic heart disease, cardiomyopathy of expansion type, etc. For this treatment method, for example, a myocardial cell sheet cultured by using a culture dish obtained by grafting poly (N-isopropylacrylamide), which is a temperature-responsive polymer, has been reported (non-patent documents 1 and 2).

### PRIOR ART LITERATURE

### Patent documents

1. Japanese patent publication No. 2006-254722
2. Japanese patent publication No. 2011-239756
3. Japanese patent publication No. 2013-247943
4. A brochure of international publication No. 2016/068266

### Non-patent documents

1. Shimizu T. et al. Fabrication of pulsatile cardiac tissue grafts using a novel 3-dimensional cell sheet manipulation technique and temperature-responsive cell culture surfaces. Circ. Res., 90, e40-e48 (2002)
2. Kawamura M. et al. Enhanced survival of transplanted human induced pluripotent stem cell-derived cardiomyocytes by the combination of cell sheets with the pedicled omental flap technique in a porcine heart. Circulation, 128 (Suppl 1), S87-S94 (2013)

### SUMMARY OF THE INVENTION

### Problems to be solved by the invention

When cells are cultured with the cell scaffold material described above, some improvement should be made as shown below. First, regarding the fiber sheet as a cell scaffold material, if the pitch which is the distance between core wires of adjacent strips of fiber is non-uniform, when cells are cultured on the fiber sheet, the cells are not held as a result, and gaps in which the cells fall off may occur at various places. In such a case, a void (hole) in which a cell does not exist in each place is generated in the obtained cell sheet, and a dense and uniform cell sheet cannot be obtained. Further, when the cell sheet is non-uniform, the quality of the cell sheet product is not constant, and the cell sheet is not able to exhibit a stable function, causing variation in quality between cell sheet products. In particular, in a cell sheet produced by using myocardial cells, when some holes exist in a myocardial cell sheet, an active potential to be lost after propagating all over to the heart is not lost when transplanted to patients with heart failure, causing reentry phenomenon by turning inside the myocardium to disturb pulsation. The heart receiving the transplantation of the myocardial cell sheet may cause abnormal pulsation.

In view of overcoming the problems of the prior art described above, it is desirable to provide a fiber sheet which can be used as a cell scaffold material and gives a stable and uniform cell sheet.

### MEANS FOR SOLVING THE PROBLEMS

For purpose of overcoming the problems of the prior art described above, the present inventor had been working diligently. As a result, he found out that an oriented fiber sheet that can be used as a cell scaffold material and gives a stable and uniform cell sheet is stably produced, and completed the invention. That is, the problems are solved by providing the following inventions of (1) to (22).
(1) An oriented fiber sheet.
(2) The fiber sheet according to (1), characterized in that the fiber forming the fiber sheet is arranged along one direction, and when the angle of the direction (orientation axis) is set to 0°, 80% or more of the strips of the fiber are arranged along angles within the range of ± 5°.
(3) The fiber sheet according to (1), characterized in that the fiber forming the fiber sheet is arranged along one direction, and when the angle of the direction (orientation axis) is set to 0°, 95% or more of the strips of the fiber are arranged along angles within the range of ± 1°.
(4) The fiber sheet according to any one of (1) to (3), wherein the diameter, in an orthogonal cross-section, of the fiber forming the fiber sheet is 1 µm to 7 µm, and the pitch of the fiber sheet is 6 µm to 60 µm.
(5) The fiber sheet according to any one of (1) to (3), wherein the diameter, in an orthogonal cross-section, of the fiber forming the fiber sheet is 2 µm to 6 µm, and the pitch of the fiber sheet is 6 µm to 50 µm.
(6) The fiber sheet according to any one of (1) to (5), which has a porosity of 10% to 60%.
(7) The fiber sheet according to any one of (1) to (6), which has a thickness of 4 µm to 70 µm.
(8) The fiber sheets according to any of (1) to (7), wherein the fiber sheets are laminated in multiple layers, and the orientation axes of the upper and lower fiber sheets contacting each other intersect at angles of 5° to 25°.
(9) The fiber sheet according to any one of (1) to (8), wherein the fiber forming the fiber sheet is prepared from a biodegradable polymer material.
(10) The fiber sheet according to (9), wherein the polymer material is one or more selected from the group consisting of copolymer of polylactic acid and polyglycolic acid (PLGA), polyglycolic acid (PGA), polybutyric acid (PLA), polyvinyl alcohol (PVA), polyethylene glycol (PEG), polyethylene vinyl acetate (PEVA), and polyethylene oxide (PEO).
(11) The fiber sheet according to (9), wherein the polymer material is copolymer of polylactic acid and polyglycolic acid (PLGA).
(12) The fiber sheet according to any one of (1) to (8), wherein the fiber forming the fiber sheet is prepared from a non-biodegradable polymer material.
(13) The fiber sheet according to (12), wherein the polymer material is one or more selected from the group consisting of polystyrene (PS), polycarbonate (PC), polymethyl methacrylate (PMMA), polyvinyl chloride, polyethylene terephthalate (PET), polyamide (PA), polymethylglutarimide (PMGI), and thermoplastic polyerterelastomer.
(14) The fiber sheet according to (12), wherein the polymer material is polystyrene.
(15) The fiber sheet according to any one of (1) to (14) for culturing cells.
(16) The fiber sheet according to (15), wherein cells are myocardial cells.
(17) A cell scaffold material comprising a fiber sheet according to any one of (1) to (14).
(18) A method for evaluating cell function, characterized by using the cell scaffold material according to (17).
(19) A production method of a fiber sheet according to any one of (1) to (14), characterized by using an electrospinning method.
(20) The production method according to (19), which uses a solution comprising a polymer material as a raw material.
(21) A cell sheet having a fiber sheet according to any one of (1) to (14) and cells.
(22) A cell sheet according to (21), wherein cells are myocardial cells.

### EFFECTS OF THE INVENTION

When using the fiber sheet of the present invention as a cell scaffold material, cells, especially myocardial cells, can be stably cultured. Compared with a myocardial cell sheet using a conventional cell scaffold material, the resulting myocardial cell sheet is improved in characteristics of extracellular potential which is determined using a microelectrode array (hereinafter abbreviated as MEA) probe, drug response, and gene expression.

### BRIEF DESCRIPTION OF THE DRAWINGS

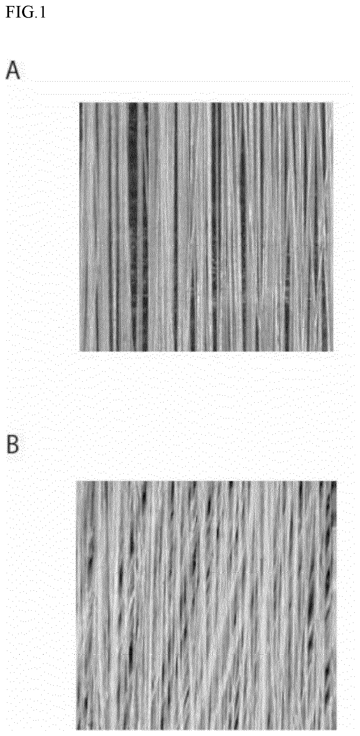

FIG.1 shows enlarged photographs of oriented fiber sheets (magnification: 2000 times). The fiber sheet in (A) is oriented fiber sheet 1, which has one layer. The fiber sheet in (B) is oriented fiber sheet 2, which has two layers stacked upon each other by changing orientation angles. (The intersection angles of orientation axes of the fiber sheet are 5° to 25°.)

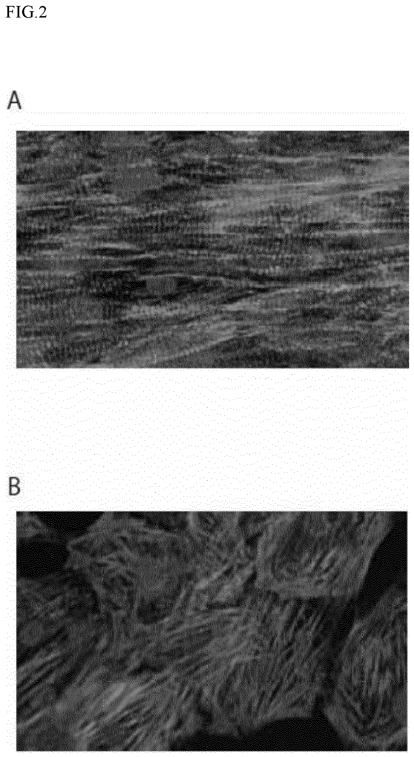

FIG.2 shows an alpha-actinin staining image and a DAPI staining image of myocardial cells cultured for 7 days using a fiber sheet according to the present invention as a cell scaffold material or a dish for cell culture, and fixed with methanol. (A) is a staining image in case of using an oriented fiber sheet 2. (B) is a staining image in case of using a dish for cell culture.

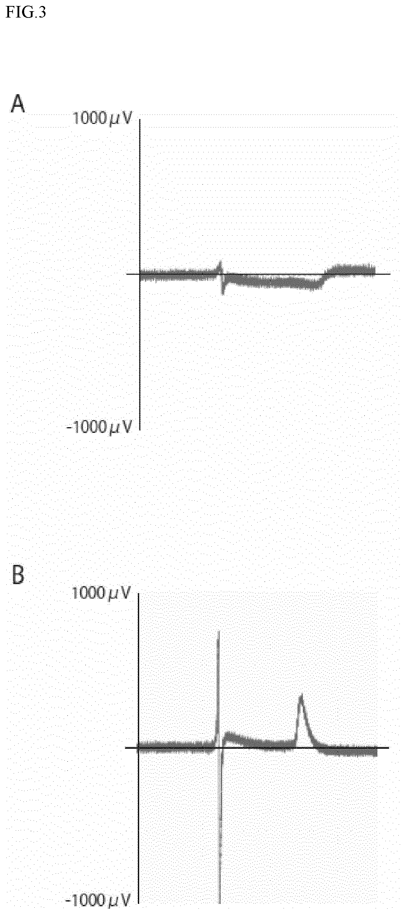

FIG.3 shows the results of the determination of extracellular potential of myocardial cell sheets cultured with oriented fiber sheets 2 with a pitch of 5 µm (A) and 10 µm (B), respectively, as a cell scaffold material using an MEA probe.

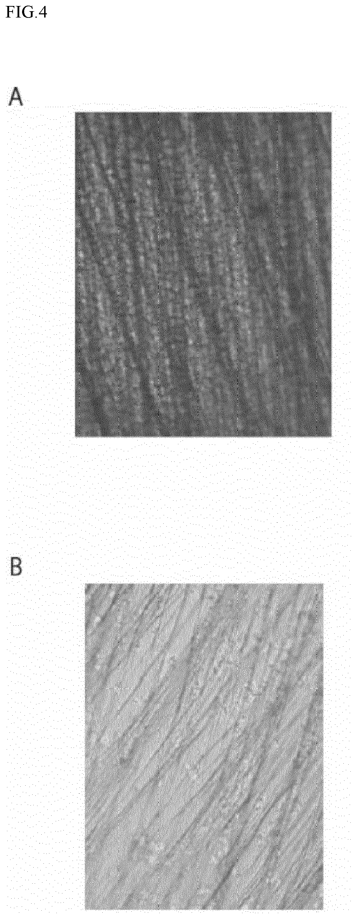

FIG.4 shows optical micrographs (magnification: 20 times) indicating the state of myocardial cell sheets cultured with oriented fiber sheets 2 with a pitch of 10 µm (A) and 70 µm (B), respectively, as a cell scaffold material.

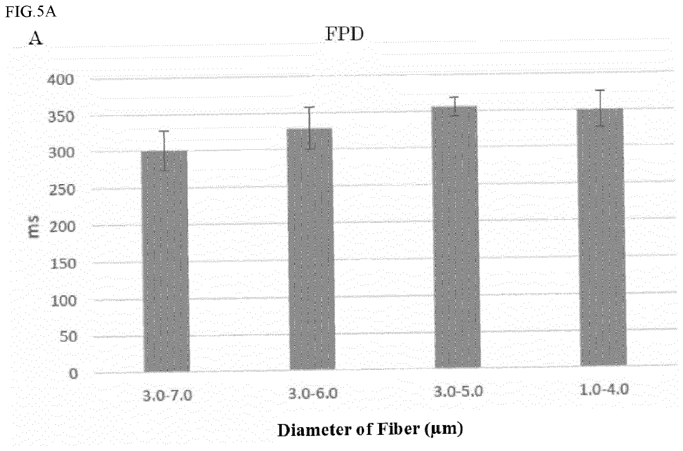

FIG.5A shows the results of the determination of extracellular potential (FPD) of myocardial cells using an MEA probe. Oriented fiber sheets 2 of different diameter of the fiber forming the fiber sheets are used as a cell scaffold material. FIG.5A shows the results of the determination of the FPD. The results are indicated by an average value ± standard deviation (n = 4).

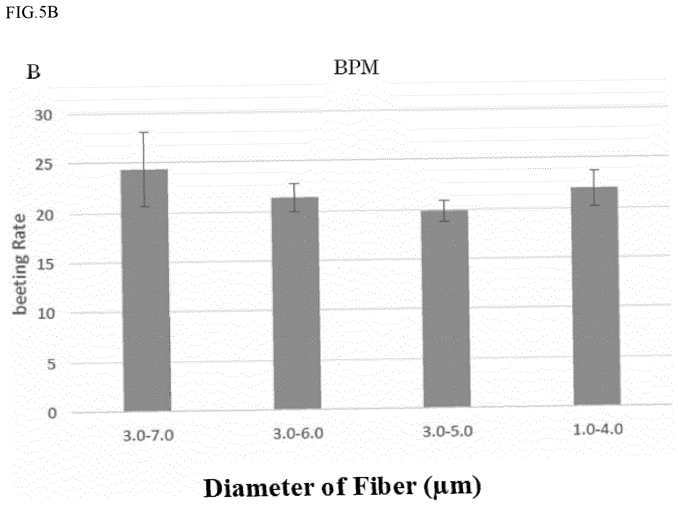

FIG.5B shows the results of the determination of extracellular potential (BPM) of myocardial cells using an MEA probe. Oriented fiber sheets 2 of different diameter of the fiber forming the fiber sheets are used as a cell scaffold material. FIG.5B shows the results of the determination of the BPM. The results are indicated by an average value ± standard deviation (n = 4).

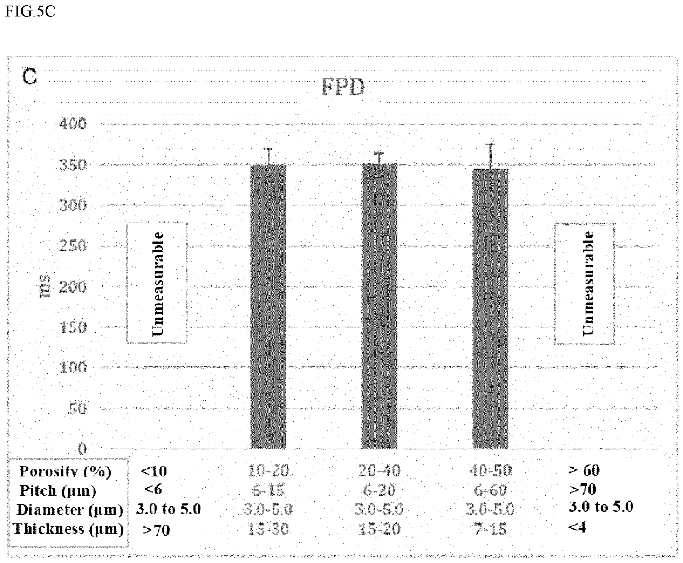

FIG.5C shows the results of the determination of extracellular potential (FPD) of myocardial cells using an MEA probe. Oriented fiber sheets 2 of different porosity, pitch and thickness of the fiber forming the fiber sheets are used as a cell scaffold material. FIG.5C shows the results of the determination of the FPD. The results are indicated by an average value ± standard deviation (n = 4).

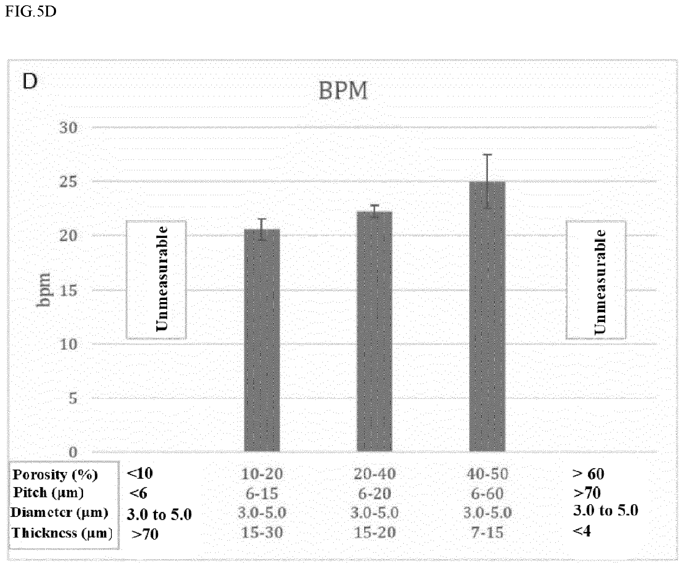

FIG.5D shows the results of the determination of extracellular potential (BPM) of myocardial cells using an MEA probe. Oriented fiber sheets 2 of different porosity, pitch and thickness of the fiber forming the fiber sheets are used as a cell scaffold material. FIG.5D shows the results of the determination of the BPM. The results are indicated by an average value ± standard deviation (n = 4).

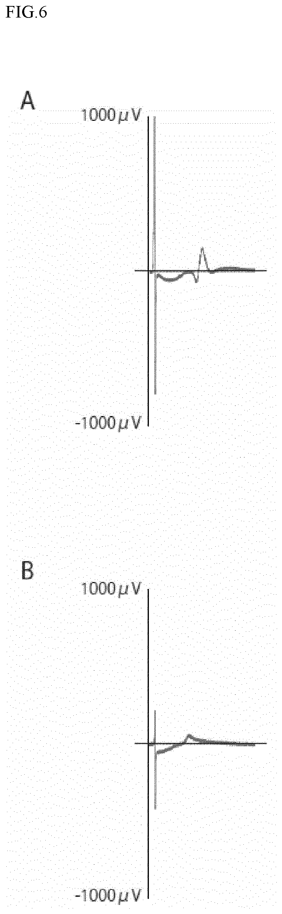

FIG.6 shows the results of the determination of extracellular potential using an MEA probe of myocardial cells. (A) shows the extracellular potential of the myocardial cells cultured with an oriented fiber sheet 2 with a pitch of 10 µm as a cell scaffold material. (B) shows the extracellular potential of the myocardial cells cultured directly on the MEA probe.

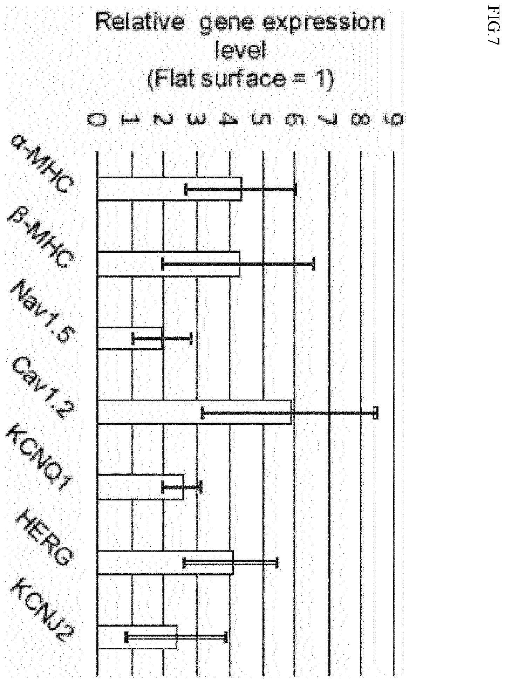

FIG.7 is a diagram comparing the gene expression level of myocardial cells cultured with an oriented fiber sheet 2 with a pitch of 10 µm as a cell scaffold material with the gene expression level of myocardial cells cultured with a dish for cell culture.

### DETAILED DESCRIPTION OF THE INVENTION

The fiber sheet according to the present invention is orientable. The oriented fiber sheet can be produced, for example, from a solution containing polymer material by an electrospinning method. The method for producing an oriented fiber sheet is not particularly limited. For example, it can be produced by using a rotary drum, spraying a solution containing a polymer material from a nozzle to the rotating surface of the drum while rotating, and winding the fiber formed on the rotary drum.

An oriented fiber sheet refers to a fiber sheet in which the strips of the fiber forming the fiber sheet are arranged along one direction. When the angle of the direction (orientation axis) is set to 0°, 80% or more, preferably 95% or more of the strips of the fiber are arranged along angles within the range of ± 5°, preferably ± 1°.

Regarding the fiber sheet for culturing cells according to the present invention, the average diameter in an orthogonal cross-section of the fiber forming the fiber sheet is, for example, in the range of 1 µm to 7 µm, preferably 2 µm to 6 µm, and more preferably 3 µm to 5 µm.

The pitch of a fiber sheet is the distance between the core wires of adjacent strips of the fiber forming the fiber sheet. Regarding the fiber sheet for culturing cells according to the present invention, the pitch is 6 µm to 60 µm, preferably 6 µm to 50 µm, and more preferably 6 µm to 30 µm.

In a fiber sheet which is one layer in the vertical direction with respect to the plane of the fiber sheet, the porosity of a fiber sheet is the ratio of the area where the fiber does not exist with respect to a fixed area of a fiber sheet plane. Regarding the fiber sheet for culturing cells according to the present invention, the porosity is 10% to 60%, preferably 15% to 50%, more preferably 20% to 40%, and further more preferably 30% to 40%.

The fiber sheet according to the present invention may be composed of either one layer of fiber sheet (single layer) in the vertical direction with respect to the fiber sheet plane, or two or more layers of fiber sheets (laminated or multilayer, for example, two layers, three layers, four layers, five layers, six layers, etc.). In the case of laminating the fiber sheets, the upper and lower fiber sheets are in contact with each other. The orientation axes of the upper and lower fiber sheets intersect at angles of 5° to 25°, preferably 10° to 20°, and more preferably 13° to 17°.

The fiber sheet according to the present invention has a thickness of, for example, 4 µm to 70 µm, preferably 5 µm to 60 µm, more preferably 5 µm to 40 µm, and further more preferably 10 µm to 30 µm.

The fiber sheet according to the present invention is a fiber sheet produced from a polymer material. The fiber forming the fiber sheet is prepared from a polymer material. The polymer material may be any material as long as it dose not exhibit cytotoxicity in contact with cells during cell culture. Biodegradable or non-biodegradable polymer materials can be used according to the purpose of use of the cell sheet obtained by culturing cells in contact with the fiber sheet.

Examples of biodegradable polymer materials include, but are not limited to, copolymers of polylactic acid and polyglycolic acid (PLGA), polyglycolic acid (PGA), polybutyric acid (PLA), polyvinyl alcohol (PVA), polyethylene glycol (PEG), polyethylene vinyl acetate (PEVA), and polyethylene oxide (PEO). PLGA is a highly safe material known to hydrolyz in-vivo, turn into lactic acid and glycolic acid, which originally present in-vivo, degrade into water and carbon dioxide, and excrete outside the body. Thus, it is particularly preferably used. Regarding the PLGA, it is possible to adjust the in-vivo decomposition rate by changing the combination ratio of PLA (polylactic acid) and PGA (polyglycolic acid).

Examples of the non-biodegradable polymer material include, but are not limited to, polystyrene (PS), polycarbonate (PC), polymethyl methacrylate (PMMA), polyvinyl chloride, polyethylene terephthalate (PET), polyamide (PA), polymethylglutarimide (PMGI), and thermoplastic polyolefin elastomers (for example, Hytreol®). Polystyrene (PS), a less cytotoxic material, is particularly preferably used.

A cell scaffold material can be molded by fixing or holding the periphery of the fiber sheet by a frame. When fixing or holding a fiber sheet to a frame, anything can be used as long as it does not affect cell culture. For example, a commercially available biocompatible adhesive, such as RVT rubber of silicone-one-liquid condensation-type (Shin-Etsu Chemical Co., Ltd., Cat. No. KE-45) can be used to adhere a fiber sheet to a frame.

The material of the frame is not particularly limited as long as it does not affect cell culture. For example, polydimethylsiloxane (PDMS), PS, polycarbonate, stainless and the like can be used.

The cell scaffold material using the fiber sheet can be arranged as it is in a dish for cell culture or at least one of the wells contained in a multi-well plate having a plurality of wells. It is also the same in cases when a fiber sheet wherein its periphery is fixed or held by a frame is used as the cell scaffold material.

Cells which can be cultured with the fiber sheet according to the present invention can be floating cells such as blood cells or lymphoid cells, or substrate-adhesive cells. Substrate-adhesive cells are preferably used. Examples of such cells include muscle cells such as myocardial cells and smooth muscle cells, hepatocytes, which are parenchymal cells of the liver, Kupffer cells, endothelial cells such as vascular endothelial cells and corneal endothelial cells, epidermal cells such as fibroblasts, osteoblasts, osteoclasts, periodontal ligament-derived cells, and epidermal keratinocytes, epithelial cells such as tracheal epithelial cells, gastrointestinal epithelial cells, cervical epithelial cells, corneal epithelial cells, nerve cells such as mammary gland cells, pericite, renal cells, knee Langerhans islet cells, peripheral nerve cells and optic nerve cells, chondrocytes, bone cells, and the like. Preferably, muscle cells such as myocardial cells and smooth muscle cells are used. These cells may be primary cultured cells directly collected from tissues or organs, or they may be passaged. Besides, an immortalized cell line may also be used. Further more, cells which can be cultured with a fiber sheet according to the present invention may be any of embryonic stem cells, which are undifferentiated cells, pluripotent stem cells such as mesenchymal stem cells with pluripotency, unipotent stem cells such as vascular endothelial progenitor cells with monopotency, and cells that have completed differentiation. Cells derived from pluripotent stem cells, such as ES cells or iPS cells, are exemplified as myocardial cells. Various methods for inducing myocardial cells from pluripotent stem cells are known. For example, the methods described in patent document 1 are exemplified. Besides, cells commercially available as myocardial cells derived from ES cells or iPS cells may also be used.

The present invention is described in detail with reference to examples. However, the present invention is not limited thereto.

### EXAMPLE 1

### Production of an oriented fiber sheet

### (1) One-direction oriented fiber sheet (hereinafter referred to as oriented fiber sheet 1)

30 wt.% PS (polystyrene, Fluka))/DMF (N, N-dimethylformamide, molecular biology grade, Wako Pure Chemical Corporation) was dissolved by rotary mix, filled in a syringe (Norm-Jevt Syringes, 5 mL, Osaka Chemicals), and installed in a nano-fiber electric field spinning apparatus (NANON-03, MEC Company Ltd.) quipped with a 25 G edge flat needle. Next, a spinning base material was stuck on the drum collector, and spinning was performed under the conditions of voltage: 8 kV to 11 kV, injection flow rate: 1.0 to 2.0 mL/hour, and drum rotation speed: 500 rpm to 2000 rpm. Thus, an oriented fiber sheet 1 was produced. An enlarged photograph of the produced oriented fiber sheet 1 obtained by photographing with a magnification of 2000 times using VHX-5000 (digital microscope, Keyence) as a photographing apparatus is shown in FIG.1A.

### (2) Two-layer oriented fiber sheet (hereinafter referred to as oriented fiber sheet 2)

30 wt.% PS (polystyrene, Fluka))/DMF (N, N-dimethylformamide, molecular biology grade, Wako Pure Chemical Corporation) was dissolved by rotary mix, filled in a syringe (Norm-Jevt Syringes, 5 mL, Osaka Chemicals), and installed in a nano-fiber electric field spinning apparatus (NANON-03, MEC Company Ltd.) quipped with a 25 G edge flat needle. Next, a spinning base material is stuck on the drum collector, and spinning was performed for the first time under the conditions of voltage: 8 kV to 11 kV, injection flow rate: 1.0 to 2.0 mL/hour, and drum rotation speed: 500 rpm to 2000 rpm.

After the first spinning, the fiber-bonded spinning base material was peeled off from the drum collector. The orientation axis was inclined by 15° and stuck again on the drum collector. Then, spinning was performed for the second time under the conditions of voltage: 8 kV to 11 kV, injection flow rate: 1.0 to 2.0 mL/hour, and drum rotation speed: 500 rpm to 2000 rpm. After the second spinning, the fiber-bonded spinning base material was peeled off from the drum collector and dried in a desiccator (autodryer desiccator, As One) for at least 3 days. Thus, an oriented fiber sheet 2 was produced. An enlarged photograph of the produced oriented fiber sheet 2 obtained by photographing with a magnification of 2000 times using VHX-5000 (digital microscope, Keyence) as a photographing apparatus is shown in FIG.1B.

### EXAMPLE 2

### Structural characteristics of the oriented fiber sheet

The results of determination of the structure of oriented fiber sheets 2 produced according to Example 1 using the methods below are shown in Table 1.

**Table 1 Structural characteristics of oriented fiber sheets 2**

| | |
|---|---|
| Diameter | 3 µm to 5 µm |
| Pitch | 6 µm to 30 µm |
| Intersect angles of orientation axes | 13° to 17° |
| Porosity | 30% to 40% |
| Sheet thickness | 10 µm to 30 µm |

### (1) Measurement of fiber diameter

VHX-5000 (digital microscope, Keyence) was used as a photographing apparatus. Five spots in an oriented fiber sheet were randomly selected and photographed at a magnification of 2000 times. Twenty strips of the fiber were randomly selected from the photographs, and their diameter, in an orthogonal cross-section of each selected fiber was measured.

### (2) Measurement of pitch

VHX-5000 (digital microscope, Keyence) was used as a photographing apparatus. Five spots in an oriented fiber sheet were randomly selected and photographed at a magnification of 2000 times. Twenty spots of the distance between each of the core wire of the adjacent strips of a specific fiber were randomly selected from the photographs, and measured.

### (3) Determination of orientation

VHX-5000 (digital microscope, Keyence) was used as a photographing apparatus. Five spots in an oriented fiber sheet were selected and photographed at a magnification of 2000 times. Twenty strips of the fiber were randomly selected from each of the photographs, and the angles to the direction along which the fiber is produced were measured.

### (4) Determination of porosity

VHX-5000 (digital microscope, Keyence) was used as a photographing apparatus. Five spots in an oriented fiber sheet were selected and photographed at a magnification of 2000 times. From each of the photographs, the intensity of the automatic area measurement was determined by the image processing software equipped in the VHX-5000, and the porosity was calculated by determining the ratio of the luminosity to the whole sheet.

### (5) Measurement of sheet thickness

The thickness of only the spinning base material and the thickness of a piece of paper to which a fiber sheet is adhered were measured randomly at five spots by using a digimatic indicator (ABS digimatic indicator ID-CX, ID-C112XBS). The sheet thickness, the difference between the two values, was calculated.

### EXAMPLE 3

Culture of myocardial cells with an oriented fiber sheet 2 as a cell scaffold material

### (1) Culture of myocardial cells

The myocardial cells derived from human iPS cells were prepared according to Example 1, seeded on an oriented fiber sheet 2 having structural characteristics shown in Table 1 or a dish for cell culture (Falcon) coated with fibronectin, and cultured for 7 days in an environment of 5% CO₂ and 37°C.

### (2) Sarcomeric orientation of the cultured myocardial cells

The myocardial cell sheet obtained by culturing for 7 days as described above was fixed with methanol. Then, alpha-actinin, an actin-binding protein, was stained using an anti alpha-actinin antibody. Further, the fluorescent staining of the nucleus with DAPI (4',6-diamidino -2-phenylindole dihydrochloride) was conducted. These staining procedures are well known to those skilled in the art. The staining images are shown in FIG.2. In either of the cases where scaffold of an oriented fiber sheet 2 (FIG.2A) and a dish for cell culture (FIG.2B) were used as a cell scaffold material, alpha-actinin dyed in green color and nuclei dyed in blue color were confirmed. When the oriented fiber sheet 2 was used, a clearly orientable myocardial cell sheet was obtained. However, it is indicated that the myocardial cell sheet obtained on the dish for cell culture was not clearly orientable. In addition, when the oriented fiber sheet 2 was used, the sarcomeric structure at organization of the myocardial cells (a structure in which alpha-actinin is present in the stripe shape relative to the orientation direction) was confirmed. Further, it was confirmed that the nucleus turned into an elliptical shape with respect to the orientation direction. It is indicated that by using the oriented fiber sheet 2 as a cell scaffold material, a structure similar with the myocardial tissue in-vivo was constructed.

### EXAMPLE 4

### Pitch of a fiber sheet and myocardial cell sheets

### (1) Determination of extracellular potential of a myocardial cell sheet using a multi-electrode array (MEA)

Oriented fiber sheets 2 with a pitch of 5 µm and 10 µm were produced according to the method of Example 1. Myocardial cells derived from human iPS cells were seeded into these fiber sheets, and cultured for 7 days in an environment of 5% CO₂ and 37°C. The resulting myocardial cell sheets were placed on a multi-electrode array (MEA) probe (MED64 system, Alpha MED Scientific Inc.) to determine the extracellular potential of myocardial cells.

FIG.3A and FIG.3B show the results of the determination of the extracellular potential of myocardial cell sheets comprising the oriented fiber sheet 2 with a pitch of 5 µm and 10 µm, respectively using an MEA. As a result, it is found that in the myocardial cell sheet cultured with the oriented fiber sheet 2 with a pitch of 10 µm, the first peak potential and the second peak potential caused by pulsation of the myocardium appear as a larger value in comparison with the myocardial cell sheet cultured with the oriented fiber sheet 2 with a pitch of 5 µm. That is, it is indicated that the myocardial cell sheet cultured with the oriented fiber sheet 2 with a pitch of 10 µm pulses more reliably. It is considered that the myocardial cells wherein adjacent cells are more closely linked can be cultured by using an oriented fiber sheet 2 with a pitch of 10 µm.

### (2) Observation of the state of the myocardial cell sheets

Oriented fiber sheets 2 with a pitch of 10 µm and 70 µm were produced according to the method of Example 1. Myocardial cells derived from human iPS cells were seeded into these fiber sheets, and cultured for 7 days in an environment of 5% CO₂ and 37°C. The state of the obtained myocardial cell sheets was observed with an optical microscope (magnification: 20 times). FIG.4A and FIG.4B show a myocardial cell sheet comprising an oriented fiber sheet 2 with a pitch of 10 µm and 70 µm, respectively. In the oriented fiber sheet 2 with a pitch of 10 µm, myocardial cells grown on the entire surface of the fiber sheet are observed (FIG.4A). On the other hand, it is found that the amount of the myocardial cells in the oriented fiber sheet 2 with a pitch of 70 µm is very small. As a cause, it is considered that the pitch of the fiber sheet is too large to the size of the cells, so that the seeded myocardial cells cannot be sufficiently held. In addition, since the distance between the myocardial cells attached to the fiber is large, the adhesion between the myocardial cells is not sufficient, and a myocardial cell sheet is not formed.

### (3) Discussion

As a result, it can be seen that regarding an oriented fiber sheet for culturing myocardial cells as a cell scaffold material, when the pitch is either too small or too large, myocardial cells can not be properly held and grown. Specifically, for the purpose of culturing myocardial cells to obtain an appropriate myocardial cell sheet, the pitch of an oriented fiber sheet is preferably 6 µm to 60 µm, more preferably 6 µm to 50 µm, and further more preferably 6 µm to 30 µm.

### EXAMPLE 5

Effects of structural characteristics of the fiber sheet forming the myocardial cell sheet on extracellular potential (FPD and BPM)
Four kinds of oriented fiber sheet 2 with different diameter of the fiber forming the fiber sheet were produced according to the method of Example 1. The values of the fiber diameter were 3 µm to 7 µm, 3 µm to 6 µm, 3 µm to 5 µm, and 1 µm to 4 µm, respectively. Myocardial cells derived from human iPS cells were seeded into these fiber sheets, and cultured for 7 days in an environment of 5% CO₂ and 37°C. The resulting myocardial cell sheets were placed on multi-electrode array (MEA) probes (MED64 system, Alpha MED Scientific Inc.). FPD (field potential duration) and BPM (beat per minute) at extracellular potential of the myocardial cells were determined (n = 4).

The results of the determination of FPD and BPM are shown in FIG.5A and FIG.5B, respectively. As a result, the FPD and BPM were excellently determined in cases of myocardial cell sheets with any fiber diameter. However, it was indicated that in cases of the myocardial cell sheets with a fiber diameter of 3 µm to 5 µm, the value of standard deviation was the smallest, and the variation of the determined values was reduced.

Next, fiber sheets with fiber diameter of 3 µm to 5 µm, and different porosity, pitch and thickness were produced. The FPD and BPM at extracellular potential were determined as above. The results are shown in FIG.5C and FIG.5D, respectively. As a result, in cases of myocardial cell sheets using the fiber sheets with the porosity of 10% to 50%, the pitch of 6 µm to 60 µm, and the thickness of 7 µm to 30 µm, the FPD and BPM were excellently determined. In particular, it was indicated that in cases of the myocardial cell sheets with the porosity of 20% to 40%, the pitch of 6 µm to 20 µm, and the thickness of 15 µm to 20 µm, the value of standard deviation was the smallest, and the variation of the determined values was reduced. On the other hand, it has been found that when the porosity of the fiber sheet is less than 10% and more than 60%, the pitch is less than 6 µm and more than 70 µm, and the thickness is less than 4 µm and more than 70 µm, an uniform myocardial cell sheet cannot be produced, and the extracellular potential cannot be determined.

As a result, by using the myocardial cell sheet of the present invention, highly accurate waveform of extracellular potential can be obtained. Therefore, highly reliable QT extension evaluation is possible.

### EXAMPLE 6

Evaluation of the function of the myocardial cell sheets cultured with oriented fiber sheets 2 Effects of the myocardial cell sheets cultured with oriented fiber sheets 2 on the extracellular potential determined with MEA, drug response and gene expression were evaluated.

### (1) Determination of extracellular potential with MEA

An oriented fiber sheet 2 with a pitch of 10 µm was produced according to the method of Example 1. Myocardial cells derived from human iPS cells were seeded on the fiber sheet, and cultured for 7 days in an environment of 5% CO₂ and 37°C. The resulting myocardial cell sheet was placed on a multi-electrode array (MEA) probe (MED64 system, Alpha MED Scientific Inc.). The extracellular potential of the myocardial cells was determined. In parallel, the myocardial cells derived from human iPS cells were seeded directly on an MEA probe, and cultured in an environment of 5% CO₂ and 37°C. Its extracellular potential was determined in the same way.

FIG.6A shows the result of determination of extracellular potential of the myocardial cell sheet produced by using an oriented fiber sheet 2 with a pitch of 10 µm. FIG.6B shows the result of determination of extracellular potential of the myocardial cells directly cultured on an MEA probe. It can be seen from FIG.6A and FIG.6B that in the myocardial cell sheet cultured with an oriented fiber sheet 2 with a pitch of 10 µm, the first peak potential and the second peak potential caused by pulsation of myocardium appear as a larger value compared with the myocardial cells directly cultured on MEA probe. That is, the potential response (S/N ratio) is remarkably improved in the myocardial cell sheet cultured with the oriented fiber sheet 2 compared with those directly cultured on MEA probe.

### (2) Drug response

An oriented fiber sheet 2 with a pitch of 10 µm was produced according to the method of Example 1. Myocardial cells derived from human iPS cells were seeded on the fiber sheet, and cultured for 7 days in an environment of 5% CO₂ and 37°C. The drug response of the resulting myocardial cells was compared with that of the myocardial cells derived from human iPS cells cultured on a flat surface of multi-electrode array (MEA) probe (MED64 system, Alpha MED Scientific Inc). Verapamil (an antiarrhythmic agent, Sigma) and dofetilide (a therapeutic agent for atrial fibrillation, Sigma) were used as target agents to study the drug response.

Table 2 shows the results of the determination of drug response to verapamil and dofetilide in the myocardial cells cultured with an oriented fiber sheet 2 and those cultured directly on MEA probe. In the myocardial cells directly cultured on MEA probe, arrest (division stop) occurred with respect to 0.3 µM verapamil, and arrhythmia occurred with respect to 0.01 µM dofetilide at a low concentration. On the other hand, in the myocardial cells cultured with an oriented fiber sheet 2, no an arrest (division stop) occurred, and no arrhythmia occurred with respect to dofetilide at a low concentration. That is, it can be seen that drug response to at least verapamil and dofetilide is improved in the myocardial cells cultured with the oriented fiber sheet 2 compared with those directly cultured on MEA probe.

**Table 2 Drug response**

| Drugs | MEA probe | Oriented fiber sheet 2 |
|---|---|---|
| Verapamil | Arrest occurred. | No arrest occurred. |
| Dofetilide | Arrhythmia occurred at a low concentration. | No arrhythmia occurred at a low concentration. |

### (3) Gene expression

Gene expression of the myocardial cells derived from human iPS cells cultured with an oriented fiber sheet 2 with a pitch of 10 µm produced by the method according to Example 1 was compared with those cultured on a dish for cell culture (Falcon). Alpha-MHC, beta-MHC, Nav1.5, Cav1.2, KCNQ1, HERG, and KCNJ2 were used as target genes to study the effect on gene expression. Gene expression was determined by using a real-time PCR method after mRNA was extracted, which is a method well known to those skilled in the art.

The results are shown in FIG.7. FIG.7 shows each of the gene expression levels of the myocardial cells cultured with an oriented fiber sheet 2 by its ratio to that of those cultured on a dish for cell culture, the level of which is set to 1.0. As is clear from FIG.7, in the myocardial cells cultured with the oriented fiber sheet 2, the expression amount for each gene is 2 to 6 times as compared with those directly cultured on the dish for cell culture. That is, in the myocardial cells cultured with the oriented fiber sheet 2, the gene expression amount of at least alpha-MHC, beta-MHC, Nav1.5, Cav1.2, KCNQ1, HERG, and KCNJ2 is increased compared with those directly cultured on a dish for cell culture.

## Claims

1. An oriented fiber sheet.

2. The fiber sheet according to claim 1, **characterized in that** the fiber forming the fiber sheet is arranged along one direction, and when the angle of the direction (orientation axis) is set to 0°, 80% or more of the strips of the fiber are arranged along angles within the range of ± 5°.

3. The fiber sheet according to claim 1, **characterized in that** the fiber forming the fiber sheet is arranged along one direction, and when the angle of the direction (orientation axis) is set to 0°, 95% or more of the strips of the fiber are arranged along angles within the range of ± 1°.

4. The fiber sheet according to any one of claims 1 to 3, wherein the diameter, in an orthogonal cross-section, of the fiber forming the fiber sheet is 1 µm to 7 µm, and the pitch of the fiber sheet is 6 µm to 60 µm.

5. The fiber sheet according to any one of claims 1 to 3, wherein the diameter, in an orthogonal cross-section, of the fiber forming the fiber sheet is 2 µm to 6 µm, and the pitch of the fiber sheet is 6 µm to 50 µm.

6. The fiber sheet according to any one of claims 1 to 5, which has a porosity of 10% to 60%.

7. The fiber sheet according to any one of claims 1 to 6, which has a thickness of 4 µm to 70 µm.

8. The fiber sheets according to any of claims 1 to 7, wherein the fiber sheets are laminated in multiple layers, and the orientation axes of the upper and lower fiber sheets contacting each other intersect at angles of 5° to 25°.

9. The fiber sheet according to any one of claims 1 to 8, wherein the fiber forming the fiber sheet is prepared from a biodegradable polymer material.

10. The fiber sheet according to claim 9, wherein the polymer material is one or more selected from the group consisting of copolymer of polylactic acid and polyglycolic acid (PLGA), polyglycolic acid (PGA), polybutyric acid (PLA), polyvinyl alcohol (PVA), polyethylene glycol (PEG), polyethylene vinyl acetate (PEVA), and polyethylene oxide (PEO).

11. The fiber sheet according to claim 9, wherein the polymer material is copolymer of polylactic acid and polyglycolic acid (PLGA).

12. The fiber sheet according to any one of claims 1 to 8, wherein the fiber forming the fiber sheet is prepared from a non-biodegradable polymer material.

13. The fiber sheet according to claim 12, wherein the polymer material is one or more selected from the group consisting of polystyrene (PS), polycarbonate (PC), polymethyl methacrylate (PMMA), polyvinyl chloride, polyethylene terephthalate (PET), polyamide (PA), polymethylglutarimide (PMGI), and thermoplastic polyerterelastomer.

14. The fiber sheet according to claim 12, wherein the polymer material is polystyrene.

15. The fiber sheet according to any one of claims 1 to 14 for culturing cells.

16. The fiber sheet according to claim 15, wherein cells are myocardial cells.

17. A cell scaffold material comprising a fiber sheet according to any one of claims 1 to 14.

18. A method for evaluating cell function, **characterized by** using the cell scaffold material according to claim 17.

19. A production method of a fiber sheet according to any one of claims 1 to 14, **characterized by** using an electrospinning method.

20. The production method according to claim 19, which uses a solution comprising a polymer material as a raw material.

21. A cell sheet having a fiber sheet according to any one of claims 1 to 14 and cells.

22. The cell sheet according to claim 21, wherein cells are myocardial cells.
